# EUROPÄISCHE PATENTSCHRIFT

(11) **EP 1 095 160 B1**
(45) Veröffentlichungstag und Bekanntmachung des Hinweises auf die Patenterteilung: **28.01.2004**
(21) Anmeldenummer: 99938217.9
(22) Anmeldetag: 08.07.1999
(51) Int. Cl.: C12P 41/00, C12P 17/10, C12P 13/00, C12P 13/02, C07C 233/52

(54) **VERFAHREN ZUR HERSTELLUNG VON (1R,4S)-2-AZABICYCLO 2.2.1 HEPT-5-EN-3-ON-DERIVATEN**
METHOD FOR PRODUCING (1R,4S)-2-AZABICYCLO 2.2.1 HEPT-5-EN-3-ON DERIVATIVES.
PROCEDE DE PREPARATION DE DERIVES DE (1R,4S)-2-AZABICYCLO 2.2.1 HEPT-5-EN-3-ONE

(30) Priorität: 09.07.1998 EP 98112719; 17.12.1998 EP 98123949
(43) Veröffentlichungstag der Anmeldung: 02.05.2001
(73) Patentinhaber: Lonza AG, 4052 Basel (CH)
(72) Erfinder: BERNEGGER-EGLI, Christine, CH-3985 Münster (CH); BRUX, Frank, CH-3942 Raron (CH); RODUIT, Jean, Paul, CH-3979 Grône (CH); WERBITZKY, Oleg, CH-3930 Visp (CH); GUGGISBERG, Yves, CH-3060 Sierre (CH)
(74) Vertreter: Ritthaler, Wolfgang, Dr.rer.nat.Dipl.-Chem.
(86) Internationale Anmeldenummer: PCT/EP1999/004814
(87) Internationale Veröffentlichungsnummer: WO 2000/003032

(56) Entgegenhaltungen:
- WO-A-98/10075
- WO-A-99/10519
- US-A- 5 688 933
- CSUK, RENE ET AL: "Biocatalytical transformations. IV. Enantioselective enzymic hydrolyses o building blocks for the synthesis of carbocyclic nucleosides" TETRAHEDRON: ASYMMETRY (1994), 5(2), 269-76 ,1994, XP002063687
- EVANS C T ET AL: "POTENTIAL USE OF CARBOCYCLIC NUCLEOSIDES FOR THE TREATMENT OF AIDS CHEMO-ENZYMATIC SYNTHESES OF THE ENANTIOMERS OF CARBOVIR" JOURNAL OF THE CHEMICAL SOCIETY, PERKIN TRANSACTIONS 1, Nr. 5, 1. Januar 1992 (1992-01-01), Seiten 589-592, XP002089871 ISSN: 0300-922X
- BRABBAN A ET AL: "STEREOSPECIFIC GAMMA-LACTAMASE ACTIVITY IN A PSEUDOMONAS FLUORESCENS SPECIES" JOURNAL OF INDUSTRIAL MICROBIOLOGY, Bd. 16, Nr. 1, 1. Januar 1996 (1996-01-01), Seiten 8-14, XP002048090 ISSN: 0169-4146
- TAYLOR S J C ET AL: "DEVELOPMENT OF THE BIOCATALYTIC RESOLUTION OF 2 -AZABICYCLO 2.2.1HEPT-5-EN-3-ONE AS AN ENTRY TO SINGLE -ENANTIOMERCARBOCYCLIC NUCLEOSIDES" TETRAHEDRON: ASYMMETRY, Bd. 4, Nr. 6, 1. Januar 1993 (1993-01-01), Seiten 1117-1128, XP002041316 ISSN: 0957-4166

## Beschreibung

Die vorliegende Erfindung betrifft ein biotechnologisches Verfahren zur Herstellung von optisch aktiven Verbindungen der allgemeinen Formeln 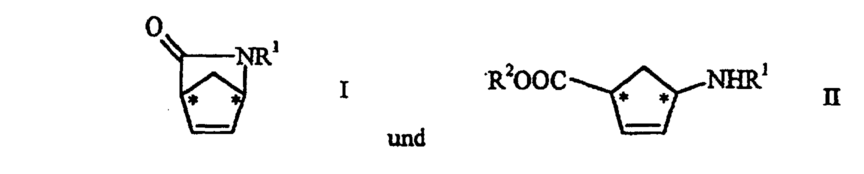 ausgehend von einem Lactam der allgemeinen Formel

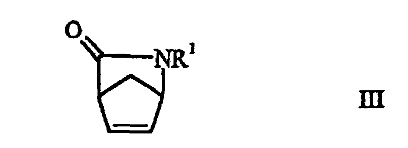

Verbindungen der Formel I wie bspw. (1R,4S)-2-Acetyl-2-azabicyclo[2.2.1]hept-5-en-3-on sind wichtige Zwischenprodukte zur Herstellung von (1R,4S)-1-Amino-4-(hydroxymethyl)-2-cyclopenten, welches wiederum ein wichtiges Zwischenprodukt zur Herstellung von carbocyclischen Nukleosiden wie z. B. Carbovir (Campbell et al., J. Org. Chem. 1995, 60, 4602-4616) ist. Verbindungen der Formel II, wie bspw. der (1S,4R)-4-Acetylamino-2-cyclopenten-1-carbonsäurepropylester, sind wichtige Zwischenprodukte zur Herstellung von (1S,4R)-1-Amino-4-(hydroxymethyl)-2-cyclopenten, welches ebenfalls ein wichtiges Zwischenprodukt zur Herstellung von carbocyclischen Nukleosiden sein kann.

Bekannt ist lediglich die chemische Herstellung von (1R,4S)-2-Acetyl-2-azabicyclo[2.2.1]hept-5-en-3-on durch Acylierung von (1R,4S)-2-Azabicyclo[2.2.1]hept-5-en-3-on (Katagiri et al., Tetrahedron Letters, 1997, 38, 1961). Gemäss diesem Verfahren kann (1R,4S)-2-Acetyl-2-azabicyclo[2.2.1]hept-5-en-3-on nur vom entsprechenden (1R,4S)-2-Azabicyclo[2.2.1]hept-5-en-3-on als Edukt erhalten werden. Dieses Edukt ist zu kostspielig.

Csuk, R. et al. [Tetrahedron: Asymmetry, 5(2), 269-276, 1994) beschreiben ein Verfahren zur Herstellung von optisch aktivem 4-Acetylamino-2-cyclopenten-1-carbonsäuremethylester ausgehend von racemischem 2-Azabicyclo[2.2.1]hept-5-en-3-on, wobei das Edukt zunächst auf chemischem Wege mit Salzsäure zur 4-Amino-2-cyclopenten-1-carbonsäure hydrolysiert und anschließend mit Methanol verestert und mit Acetanhydrid acetyliert wird. Der so erhaltene racemische 4-Acetylamino-2-cyclopenten-1-carbonsäuremethylester wird anschließend zum Erhalt des optisch aktiven Isomers mit einer Lipase aus *Candida cylindracea* hydrolysiert.

Evans, T. et al. (J. Chem. Soc. Perkin Trans. 1, 5(1), 589-592, 1992) beschreiben die Hydrolyse von racemischem 2-Azabicyclo[2.2.1]hept-5-en-3-on mittels Mikroorganismen der Spezies *Pseudomonas solanacearum*, wobei optisch aktive (1R,4S)-4-Amino-2-cyclopenten-1-carbonsäure und optisch aktives (-)-2-Azabicyclo[2.2.1]hept-5-en-3-on gebildet wird. Letzteres wird zur Herstellung von (1S,4R)-4-Acetylamino-2-cyclopenten-1-carbonsäuremethylester chemisch hydrolysiert, verestert und acetyliert.

Brabban, A. et al. (J. Industrial Microbiology, 16(1), 8-14, 1996) und US-A-5 688 933 offenbaren ein Verfahren zur Herstellung von optisch aktivem 2-Azabicyclo[2.2.1]hept-5-en-3-on und von optisch aktiver 4-Amino-2-cyclopenten-1-carbonsäure mittels Lactamasen.

Aufgabe der vorliegenden Erfindung war es, ein Verfahren zur Herstellung von Verbindungen der allgemeinen Formel I und II bereitzustellen, welche aus leicht erhältlichem, billigem, Ausgangsmaterial in guter Enantiomerenreinheit hergestellt werden können.

Diese Aufgabe wird durch die biotechnologischen Verfahren gemäss den Ansprüchen 1 und 4 gelöst.

Das erfindungsgemässe Verfahren zur Herstellung der Verbindungen der allgemeinen Formeln 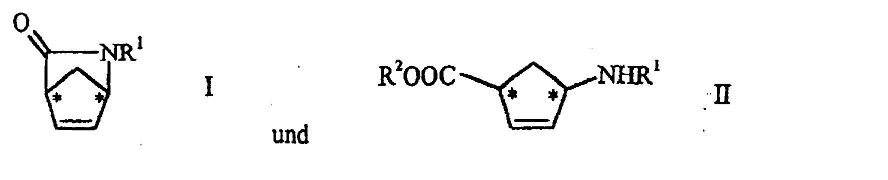 worin R¹ Acyl, Alkoxycarbonyl oder Aryloxycarbonyl und R² ein Wasserstoffatom oder C₁₋₁₀-Alkyl bedeutet, erfolgt mittels einer Hydrolase in Gegenwart eines Nucleophils bei einem innerhalb +/- 0,5 pH-Einheiten durch Zugabe einer Base konstant gehaltenen pH-Wert ausgehend von einem racemischem Lactam der Formel

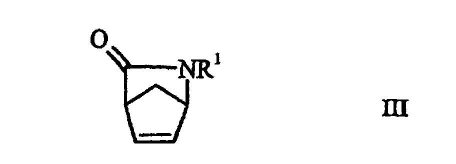

Das Ausgangsmaterial, das Lactam der allgemeinen Formel III (Substrat) kann beispielsweise gemäss Taylor et al., (Tet. Asymmetry. 4, 1993, 1117) hergestellt werden.

C₁₋₁₀-Alkyl ist linear oder verzweigt sowie substituiert oder unsubstituiert. Beispiele für C₁₋₁₀-Alkyl sind Methyl, Ethyl, Propyl, Butyl, Isobutyl, *tert*-Butyl, Isopropyl, Pentyl, Hexyl, Heptyl, Octyl, Nonyl oder Decyl und seine Isomere sowie Chlormethyl, Brommethyl, Dichlormethyl, Dibrommethyl, Chlorpropyl, Brombutyl.

Acyl bedeutet Alkanoyl oder Arylcarbonyl. Alkanoyl ist zweckmässig C₁₋₄-Alkanoyl, das substituiert oder unsubstituiert sein kann. Unter substituiertem C₁₋₄-Alkanoyl wird im folgenden mit einem oder mehreren Halogenatomen substituiertes verstanden. Beispiele für C₁₋₄-Alkanoyl sind Acetyl, Propionyl, Butyryl, Chloracetyl, Bromacetyl, Dichloracetyl. Arylcarbonyl ist zweckmässig Benzylcarbonyl oder Phertylcarbonyl, substituiert oder unsubstituiert.

Alkoxycarbonyl ist zweckmässig C₁₋₄-Alkoxycarbonyl wie Methoxycarbonyl, Ethoxycarbonyl, Propoxycarbonyl, Butoxycarbonyl oder tert-Butoxycarbonyl (BOC). Arlyoxycarbonyl ist zweckmässig Benzyloxycarbonyl oder Phenyloxycarbonyl. Vorzugsweise bedeutet R¹ C₁₋₄-Alkanoyl oder C₁₋₄-Alkoxycarbonyl insbesondere Acetyl oder Ethoxycarbonyl.

Als Hydrolasen können Proteasen oder Lipasen, vorzugsweise Proteasen wie Serinproteasen eingesetzt werden. Als Serinproteasen können bspw. Chymotrypsine, Trypsine und Subtilisine (bakterielle Serinproteasen) eingesetzt werden. Als Subtilisine können käufliche Subtilisine wie Subtilisin A, Subtilisin B, Alcalasen, ALK-Enzyme, Bacillopeptidase A, Bacillopeptidase B, Bioprasen, Colistinasen, Esperasen, Genenase I, Kazusase, Maxacal, Maxatasen, Nagarsen, Peptidasen, Protease S, Protease VIII, Protease XXVII, Proteinasen, wie die alkaline Proteinase von Bacillus subtilis oder Aspergillus oryzae, Proteinase K von Tritirachium album, Savinasen, Subtilopeptidasen, Superasen, Thermoasen verwendet werden. Vorzugsweise wird die Biotransformation mittels Savinasen durchgeführt. Als Savinasen sind Savinase 12 Type W™, Savinase 16.0L Type-EX™, Savinase 32.0L Type - EX™, Savinase 4.0T Type W™ und Savinase 8.0L™ geeignet. Als Lipase kann bspw. Lipase aus Candida antarctica verwendet werden.

Werden als Hydrolasen Proteasen, wie Savinasen, Proteasen von Bacillus subtilis, Proteasen von Aspergillus oryzae, Proteinase K von Tritirachium album eingesetzt, wird zweckmässig im racemischem Lactam der Formel III das (1S,4R)-Enantiomere in die entsprechende Verbindung der allgemeinen Formel II hydrolysiert, wobei das (1R,4S)-Enantiomere der allgemeinen Formel I anfällt. Werden als Hydrolasen Lipasen wie Lipase von Candida antarctica eingesetzt, wird zweckmässig im racemischen Lactam der Formel III das (1R,4S)-Enantiomere in die entsprechende Verbindung der allgemeinen Formel II hydrolysiert, wobei das (1S,4R)-Enantiomere der allgemeinen Formel I anfällt.

Als Nucleophil können Wasser oder C₁₋₁₀-Alkohole eingesetzt werden. Als C₁₋₁₀-Alkohole sind geeignet Methanol, Ethanol, Propanol, Isopropanol, Butanol, tert-Butanol, Isobutanol, Pentanol, Hexanol, Heptanol, Oktanol, Nonanol oder das Decanol. Wird als Nucleophil ein C₁₋₁₀-Alkohol eingesetzt, wird wie fachmännisch bekannt der entsprechende Ester der allgemeinen Formel II (R²=C₁₋₁₀-Alkyl) gebildet. Wird als Nucleophil Wasser eingesetzt, wird selbstverständlich die entsprechende Säure der allgemeinen Formel II (R²=H) gebildet.

Abhängig von der Hydrolase und dem Substrat (Lactam der Formel III) wird die Biotransformation zweckmässig zwischen pH 5 und 12, vorzugsweise zwischen pH 6 und 8, durchgeführt. Erfindungsgemäss wird dabei bei einer gegebenen Hydrolase und einem gegebenen Substrat der pH-Wert innerhalb +/- 0,5 pH-Einheiten durch Zugabe einer Base konstant gehalten. Wird z.B. als Substrat racemisches 2-Acetyl-2-azabicyclo[2.2.1]hept-5-en-3-on (R¹ = Acetyl) und als Hydrolase Savinase eingesetzt, wird der pH-Wert vorzugsweise zwischen pH 7,0 und pH 7,5 konstant gehalten.

Als Base kann eine anorganische oder organische Base eingesetzt werden. Als anorganische Basen sind z. B. KOH, NaOH, geeignet. Als organische Base kann beispielsweise Triethanolamin gelöst in einem organischen Lösungsmittel geeignet sein.

Wird als Nucleophil einer der oben beschriebenen Alkohole verwendet, kann als Base das entsprechende Alkoholation dienen.

Die Reaktionstemperatur kann in einem Bereich von 10 bis 60 °C, vorzugsweise von 15 bis 40 °C, liegen.

Zweckmässig wird die Biotransformation in Wasser, einer Pufferlösung, einem C₁₋₁₀-Alkohol oder in einem Gemisch von diesen mit einem aprotischen organischen Lösungsmittel durchgeführt. Als aprotische organische Lösungsmittel sind bspw. Ether, aromatische Kohlenswasserstoffe geeignet. Als Ether können Tetrahydrofuran, Dioxan oder tert-Methylbutyl-ether verwendet werden. Als aromatische Kohlenwasserstoffe sind Toluol und Benzol geeignet. Als Pufferlösungen können z. B. niedermolare wie 10-100mM Natrium- oder Kaliumphosphatpuffer, Hepes-Puffer verwendet werden. Als C₁₋₁₀-Alkohole können die zuvor beschriebenen eingesetzt werden.

Die Biotransformation kann auch derart durchgeführt werden, dass das Lactam der allgemeinen Formel III als Lösungsmittel dient. Dann wird zweckmässig die Biotransformation in Gegenwart der Hälfte der stöchiometrischen Mengen an Wasser oder des entsprechenden Alkohols durchgeführt.

Je nach Lösungsmittel kann die Biotransformation in einem Zweiphasen- oder Einphasensystem durchgeführt werden. Zweckmässig wird die Biotransformation in einem Einphasensystem durchgeführt.

Nach einer üblichen Umsetzungszeit von wenigen Stunden werden dann abhängig von dem gewählten Ausgangsmaterial die gewünschten optisch aktiven Verbindungen der allgemeinen Formeln I und II in hervorragender Ausbeute und Enantiomerenreinheit erhätem Die bevorzugten Ausgangsmaterialien sind racemisches 2-Acetyl-2-azabicyclo[2.2.1]hept-5-en-3-on (R¹=Acetyl) und racemisches 2-Ethoxycarbonyl-2-azabicyclo[2.2.1.]hept-5-en-3-on. Die bevorzugten Produkte der Verbindungen der allgemeinen Formel I sind (1R,4S)-2-Acetyl-2-azabicyclo[2.2.1]hept-5-en-3-on (R¹ = Acetyl) und (1R,4S)-2-Ethoxycarbonyl-2-azabicyclo[2.2.1]hept-5-en-3-on (R¹ = Ethoxycarbonyl). Die bevorzugten Verbindungen der allgemeinen Formel II sind (1S,4R)-4-Acetylamino-2-cyclopenten-1-carbonsäure (R¹ = Acetyl, R² = H), (1S,4R)-4-Ethoxycarbonyl-2-cyclopenten-1-carbonsäure (R¹ = Ethoxycarbonyl, R² = H), (1S,4R)-4-Acetylamino-2-cyclopenten-1-carbonsäuremethylester (R¹ = Acetyl, R² = CH₃), (1S,4R)-4-Acetylamino-2-cyclopenten-1-carbonsäurebutylester (R¹ = Acetyl, R² = C₄H₉), (1S,4R)-4-Acetylamino-2-cyclopenten-1-carbonsäureethylester (R¹ = Acetyl, R² = C₂H₅) und (1S,4R)-4-Acetylamino-2-cyclopenten-1-carbonsäurepropylester (R¹ = Acetyl, R² = C₃H₇). Die (1S,4R)-4-Acetylamino-2-cyclopenten-1-carbonsäure-C₂-₁₀-alkylester, vorzugsweise der (1S,4R)-4-Acetylamino-2-cyclopenten-1-carbonsäureethylester und der (1S,4R)-4-Acetylamino-2-cyclopenten-1-carbonsäurepropylester, der allgemeinen Formel II sind in der Literatur noch nicht beschrieben und demzufolge ebenfalls Bestandteil der Erfindung.

Ein weiterer Bestandteil der Erfindung ist die Weiterumsetzung, die Reduktion der Verbindung der allgemeinen Formel I, zu einem optisch aktiven 1-Amino-4-(hydroxymethyl)-2-cyclopenten-Derivat, insbesondere zu einem (1R, 4S)-1-Amino-4-(hydroxymethyl)-2-cyclopenten-Derivat der allgemeinen Formel 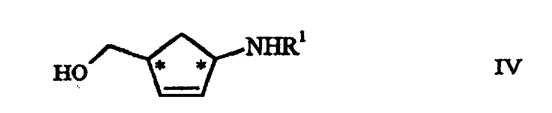 worin R¹ die genannte Bedeutung hat.

Zweckmässig wird die Reduktion mit binären oder komplexen Metallhydriden der Bor- oder Aluminiumgruppe durchgeführt wie mit Alkalimetall-, Erdalkalimetallborhydriden, Alkalimetall-, Erdalkalimetallaluminiumhydriden.

Als binäre Alkalimetall- oder Erdalkalimetallborhydride können NaBH₄, LiBH₄, KBH₄, NaAlH₄, LiAlH₄, KAlH₄, Mg(BH₄)₂, Ca(BH₄)₂, Mg(AlH₄)₂, Ca(AlH₄)₂ verwendet werden. Komplexe Metallhydride der Bor- oder Aluminiumgruppe können die allgemeine Formel M¹ M²HₙLₘ haben, worin n eine ganze Zahl von 1 bis 4 ist und m eine ganze Zahl von 4 bis 4 - n ist, M¹ ein Alkalimetallatom, M² Bor oder Aluminim bedeutet und L C₁₋₄-Alkyl, C₁₋₄-Alkenyl, C₁₋₄-Alkoxy, CN oder ein Amin ist, oder die komplexen Metallhydride können die allgemeine Formel M²HₒLₚ haben, worin M² die genannte Bedeutung hat und o eine ganze Zahl von 0 bis 3 ist und p eine ganze Zahl von 3 bis 3 - o ist. Als M¹ M² HₙLₘ können LiBH (C₂H₅)₃, LiBHₓ (OCH₃)₄₋ₓ, worin x eine ganze Zahl von 1 bis 3 bedeutet, LiAlH(OC(CH₃)₃)₃, NaAlH₂(OC₂H₄OCH₃)₂, NaAlH₂(C₂H₅)₂ oder NaBH₃CN eingesetzt werden. Vorzugsweise wird die Reduktion mit einem Metallborhydrid wie Natriumborhydrid durchgeführt.

Zweckmässig werden die Metallhydride in einem molaren Verhältnis von 0,5 bis 1 pro mol der Verbindung der allgemeinen Formel I eingesetzt.

Zweckmässig wird die Reduktion unter Inertgasatmosphäre wie beispielsweise unter Argonoder Stickstoffatmosphäre durchgeführt.

Die Reduktion kann bei einer Temperatur von -10 bis 30 °C, vorzugsweise bei einer Temperatur von 0 bis 10°C, durchgeführt werden.
Als Lösungsmittel sind für die Reduktion sekundäre oder tertiäre Alkohole geeignet. Als sekundärer Alkohol kann bspw. 2-Butanol und als tertiärer Alkohol kann bspw.
tert. Amylalkohol eingesetzt werden. Vorzugsweise wird ein sekundärer Alkohol eingesetzt.

Die Weiterumsetzung, die Hydrolyse der optisch aktiven 1-Amino-4-(hydroxymethyl)-2-cyclopenten-Derivate der Formel IV zu den entsprechenden optisch aktiven 1-Amino-4-(hydroxymethyl)-2-cyclcopentenen bzw. deren Salze, der Formel 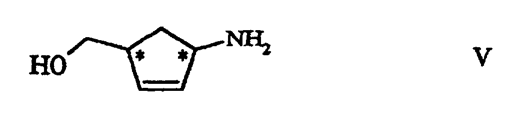 mit einem Erdalkali-, Alkalimetallhydroxid oder mit einer Mineralsäure ist ebenfalls Bestandteil der Erfindung. Insbesondere wird das (1R,4S)-1-Amino-4-(hydroxymethyl)-2-cyclopenten-Derivat zum (1R,4S)-1-Amino-4-(hydroxymethyl)-2-cyclopenten hydrolysiert.

Als Alkalimetallhydroxid ist Lithium-, Natrium- oder Kaliumhydroxid geeignet. Als Erdalkalimetallhydroxid kann bspw. Bariumhydroxid eingesetzt werden. Als Mineralsäuren sind Halogenwasserstoffsäuren wie bspw. Salzsäure oder Bromwasserstoffsäure geeignet.

Zweckmässig wird die Hydrolyse bei einer Temperatur von 50 bis 120 °C, vorzugsweise von 90 bis 100°C, durchgeführt.

Als Salze des (1R,4S)-1-Amino-4-(hydroxymethyl)-2-cyclopentens (Formel V) sind dessen Hydrohalogenidsalze wie Hydrochloride oder Hydrobromide geeignet.

### Beispiele

### Beispiel 1

### Herstellung von (1R,4S)-1-Amino-4-(hydroxymethyl)-2-cyclopenten ausgehend von racemischem (±)-2-Acetyl-2-azabicylco[2.2.1]hept-5-en-3-on

### 1.1 Herstellung von (1R,4S)-2-Acetyl-2-azabicyclo[2.2.1]hept-5-en-3-on

### 1.1.1 mittels Savinase in einem Gemisch von Natriumphosphat-Puffer und Tetrahydrofuran

5 ml Tetrahydrofuran wurden mit 3, 84 ml 20 mM Natrium-Phosphatpuffer pH 7 und 1, 16 ml Savinase 16.0 L Type EX, Novo Nordisk (16 KNPU/g, Kilo Novo Protease Units) gemischt. Zu dem Ansatz wurden 417 µl, (+/-) 2-Acetyl-2-azabicyclo[2.2.1]hept-5-en-3-on (330 mmol/l) gegeben. Die Reaktion wurde mit einem Magnetrührer gemischt und mittels eines Wasserbades bei 30°C gehalten. Der pH-Wert wurde durch eine pH-Regelung mit 1M NaOH konstant bei pH 7 gehalten. Proben wurden periodisch entnommen und mit HPLC auf Gehalt und Enantiomerenüberschuss analysiert (Chriralpak AD, Daicel Chemical Ltd. (0,46 x 25 cm), isokratisch bei Raumtemperatur, Fluss 1 ml/min mit n-Heptan (Ethanol 2%, Detektion bei 215 nm)). Insgesamt wurden dabei 1,25 ml NaOH verbraucht. Nach 120 Minuten war 50% der eingesetzten Menge an (+/-) 2-Acetyl-2-azabicyclo[2.2.1]hept-5-en-3-on zur entsprechenden Säure hydrolysiert. Die Gehaltsbestimmung erfolgte mit achiraler GC. Die Analytik wurde mit Gaschromatographie wie folgt durchgeführt: Kapillar-Säule: HP -5 (5% Phenylmethylsiloxan), Temperaturgradient 100 °C - 260 °C, die Proben wurden für die Analyse in Tetrahydrofuran 1:1 verdünnt.

### 1.1.2 in Wasser

**1.1.2.1** Zu 419,25 ml (+/-) 2-Acetyl-2-azabicyclo[2.2.1]hept-5-en-3-on wurden 60 ml H₂O und 35 ml Savinase gemäss Beispiel 1.1.1 gegeben. Der pH-Wert wurde mit 7,5 N NaOH auf 7,5 gehalten, der 11-Applikon Fermenter wurde mit 400 Upm gerührt. Gemäss Beispiel 1.1.1 wurden Proben periodisch entnommen und analysiert. Nach 45 h wurde ein ee-Wert von 99% gemessen.
   Der Ansatz wurde über ein Whatman GF/F Filter abgenutscht und der Filterkuchen wurde 2 mal mit 100 ml und 1 mal mit 50 ml Butylacetat gewaschen. Die wässrige Phase wurde 2 mal mit 200 ml Butylacetat ausgeschüttelt. Die organische Phase wurde am Rotavap eingeengt. 144,8 g Produkt mit einem ee-Wert von > 98% wurden erhalten, was einer Ausbeute von 31% bez. des Racemats entsprach. Die Analytik wurde gemäss Beispiel 1.1.1 durchgeführt.
1.1.2.2 Zu 486,3 g (+/-) 2-Acetyl-2-azabicyclo[2.2.1]hept-5-en-3-on (Gehalt: 95,4%) wurden 60 ml H₂O und 35 ml Savinase gemäss Beispiel 1.1.1 gegeben. Der pH-Wert wurde mit 7,5 N NaOH auf 7,5 gehalten, der 11-Applikon Fermenter wurde mit 400 Upm gerührt. Gemäss Beispiel 1.1.1 wurden Proben periodisch entnommen und analysiert. Nach 45 h wurde ein ee-Wert von >98% gemessen. Insgesamt wurden 174,6 ml 7,5 N NaOH verbraucht.
   Der Ansatz wurde über ein Whatman GF/F Filter abgenutscht und der Filterkuchen wurde 2 mal mit 100 ml und 1 mal mit 50 ml Butylacetat gewaschen. Die wässrige Phase wurde 2 mal mit 200 ml Butylacetat ausgeschüttelt. Die organische Phase wurde am Rotavap eingeengt. 144,8 g Produkt mit einem ee-Wert von > 98% (Gehalt: 92,5%) wurden erhalten, was einer Ausbeute von 29% bez. des Racemats entsprach. Die Analytik wurde gemäss Beispiel 1.1.1 durchgeführt.

### 1.1.3 in Methanol

72,3 ml (+/-) 2-Acetyl-2-azabicyclo[2.2.1]hept-5-en-3-on (Gehalt 95,4%) wurden mit 8,3 ml Savinase gemäss Beispiel 1.1.1 und 19,4 ml Methanol versetzt. Das Reaktionsgemisch wurde über einen Zeitraum von 24 h bei 30°C gerührt. Der pH blieb dabei konstant bei 7,2. Proben wurden nach Beispiel 1.1.1 entnommen. Nach 25 h wurde ein ee-Wert von > 98% erreicht und die Reaktion wurde beendet. Die analytische Ausbeute betrug 42% bez. des Racemats. Dabei bildete sich in stöchiometrischen Mengen der entsprechende Methylester (R² = CH₃) der Verbindung der allgemeinen Formel II, was mit GC-MS bewiesen wurde. Die Analytik wurde gemäss Beispiel 1.1.1 durchgeführt.

### 1.1.4 in Butanol

72,3 ml (+/-) 2-Acetyl-2-azabicyclo[2.2.1]hept-5-en-3-on (Gehalt 95,4%) wurden mit 76,86 ml 1-Butanol und 8,3 ml Savinase gemäss Beispiel 1.1.1 versetzt. Das Reaktionsgemisch wurde über einen Zeitraum von 22 h gemessen. Nach dieser Zeit wurde ein ee-Wert von > 98% mit HPLC bestimmt. Der pH-Wert wurde mit 4 N NaOH bei pH 7,5 konstant gehalten. Proben wurden gemäss Beispiel 1.1.1 entnommen. Die Reaktionstemperatur lag bei 30°C. Es bildete sich freie Säure (R² = H) der Verbindung der allgemeinen Formel II, was anhand von HPLC bewiesen wurde und der Butylester (R² = C₄H₉) der Verbindung der allgemeinen Formel II, was anhand von GC-MS bewiesen wurde. Eine analytische Ausbeute von 34,8% bez. des Racemats wurde erreicht.

### 1.1.5 mittels Savinase in 1-Propanol

**1.1.5.1** 242g (+/-) 2-Acetyl-2-azabicyclo[2.2.1]hept-5-en-3-on, 168,6 ml 1-Propanol und 28 ml Savinase gemäss Beispiel 1.1.1 wurden bei 30°C und bei einem pH von 7,0 (eingestellt mit 4 N NaOH) inkubiert. Proben wurden gemäss Beispiel 1.1.1 entnommen. Nach 24 h wurde der ee-Wert des Produktes zu % ee = 99% ermittelt. Die analytische Ausbeute betrug 47%. Anschliessend wurden zu dieser Mischung (459 ml) 100 ml Toluol hinzugegeben und das Propanol unter reduziertem Druck abgedampft. Dann wurde die Lösung zweimal mit Toluol (250 ml) und Wasser (100 ml) extrahiert. Toluol wurde abgedampft und das Produkt destilliert. Es wurden 113,9 g (0,69 mol) Produkt (Reinheit 93%, ee = 99%) entsprechend einer Ausbeute von 45,7% bez. des Racemats erhalten. Die Analytik wurde gemäss Beispiel 1.1.1 durchgeführt.
**1.1.5.2** 208g (+/-) 2-Acetyl-2-azabicyclo[2.2.1]hept-5-en-3-on (Gehalt: 95,4%), 168,6 ml 1-Propanol und 23 ml Savinase gemäss Beispiel 1.1.1 wurden bei 30°C und bei einem pH von 7,2 (eingestellt mit 4 N NaOH) im Erlenmeyerkolben inkubiert. Nach 30 h wurde der ee-Wert des Produktes zu % ee = > 98% ermittelt. Anschliessend wurden zu dieser Mischung (459 ml) 100 ml Toluol hinzugegeben und das Propanol unter reduziertem Druck abgedampft. Dann wurde die Lösung zweimal mit Toluol (250 ml) und Wasser (100 ml) extrahiert. Toluol wurde abgedampft und das Produkt destilliert (12 mbar, 85 - 95°C). Es wurden 79,13 g (0,69 mol) Produkt (Reinheit 93%, ee > 98%) entsprechend einer Ausbeute von 37% bez. des Racemats erhalten. Die Analytik wurde gemäss Beispiel 1.1.1 durchgeführt.
**1.1.5.3** 2,77 kg (+/-) 2-Acetyl-2-azabicyclo[2.2.1]hept-5-en-3-on (Gehalt 95,8%), 1,351 1-Propanol und 355,5 ml Savinase gemäss Beispiel 1.1.1. wurden bei 40°C und einem pH-Wert = 7,4 (eingestellt durch Zugabe von 92g 4N NaOH) in einem 5 1 Rührreaktor umgesetzt. Hierbei wurde der pH-Wert durch Zugabe von 4N NaOH bei pH = 7,4 konstant gehalten. Nach 14 h wurde das Reaktionsgemisch auf Raumtemperatur abgekühlt und der pH-Wert der Lösung durch Zugabe von 20%iger Schwefelsäure auf pH = 7, 0 eingestellt. Nach Zugabe von 1,15 1 Toluol wurden die Phasen getrennt und die organische Phase im Vakuum destilliert (Produktfraktion bei T=72-76°C, p=1 mbar). Es wurden 1,07 kg Produkt (Gehalt 91%, ee = 98,4%) entsprechend einer Ausbeute von 36,5% erhalten. Der Gehalt des Produkts wurde mittels GC auf einer Optima5-Säule (Macherey-Nagel, Deutschland) bestimmt. Der ee des Produkts wurde mittels GC auf einer chiralen LipodexE-Säule (Macherey-Nagel, Deutschland) bestimmt.

### 1.1.5.4 Isolation von (1S,4R)-Acetylamino-2-cyclopenten-1-carbonsäurepropylester

345 g des Blasenrests aus Beispiel 1.1.5.3 wurden mit 250 ml Esssigsäureethylester und 11 Hexan versetzt und das Gemisch wurde auf Rückflusss erhitzt. Es bildeten sich zwei Phasen, die obere Phase wurde abgetrennt und langsam auf 0°C abgekühlt. Der Niederschlag wurde abfiltriert und bei 40°C im Vakuum getrocknet. Es wurden 85,6 g farbloses Produkt erhalten.

Der ee-Wert wurde mittels GC auf einer chiralen Hydrodex-β-PM-Säule (Macherey-Nagel, Deutschland) bestimmt, ee = 93%. α_{D,20°C}(c=1, MeOH)= 79,3°
¹H-NMR(CDCl₃):
5,91 (br,1H)
5,89 (s, 2H)
5,07 (m, 1H)
4.07 (t, 2H)
3,50 (m, 1H)
2,46 (m, 1H)
1,96 (s, 3H)
1,90 (m; 1H)
1,67 (m 2H)
0,96 (t, 3H)

### 1.1.6 mittels Protease von Bacillus subtilis in Phosphat-Puffer

**1.1.6.1** 25 mg Bacillus subtilis Protease (Fluka 82490), 0,45 ml Phosphat-Puffer (100 mM, pH 7,5), 0,5 ml n-Propanol und 0,05 ml (+/-) 2-Acetyl-2-azabicyclo[2.2.1] hept-5-en-3-on wurden bei 30 °C (+/- 2 °C) inkubiert. Der pH Wert wurde durch manuelle Zugabe von 1 N NaOH bei 7,5 gehalten, wobei Schwankungen von +/- 0,5 pH erreicht wurden. Proben wurden gemäss Beispiel 1.1.1 entnommen. Nach 6 h war alles (+/-) 2-Acetyl-2-azabicyclo[2.2.1] hept-5-en-3-on umgesetzt. 1,5 h nach Inkubation war der Enantiomerenüberschuss von (-) 2-Acetyl-2-azabicyclo[2.2.1] hept-5-en-3-on >98%. Die nicht isolierte Ausbeute betrug 12% bez. des Racemats. Gehalt und Enantiomerenüberschuss wurden wie in Beispiel 1 beschrieben bestimmt.
**1.1.6.2** 125 mg Bacillus subtilis Protease (Fluka 82490), 2,25 ml Phosphat-Puffer (100 mM, pH 7,5), 2,5 ml n-Propanol und 0,25 ml (+/-) 2-Acetyl-2-azabicyclo[2.2.1] hept-5-en-3-on (Gehalt: 95,4%) wurden bei 30 °C (+/- 2 °C) inkubiert. Der pH Wert wurde durch manuelle Zugabe von 1 N NaOH bei 7,5 gehalten, wobei Schwankungen von +/- 0,5 pH erreicht wurden. Proben wurden gemäss Beispiel 1.1.1 entnommen. Nach 6 h war 90% des (+/-) 2-Acetyl-2-azabicyclo[2.2.1] hept-5-en-3-on umgesetzt. 1,5 h nach der Inkubation war der Enantiomerenüberschuss von (-) 2-Acetyl-2-azabicyclo[2.2. 1] hept-5-en-3-on >98%. Die nicht isolierte Ausbeute betrug dann 12% bez des Racemats.

### 1.1.7 mittels Protease von Aspergillus oryzae in Phosphat-Puffer

**1.1.7.1** 25 mg Aspergillus oryzae (Sigma P-4032, 3.5 Units/mg), 0,45 ml Phosphat-Puffer (100 mM, pH 7,5), 0,5 ml n-Propanol und 0,05 ml (+/-) 2-Acetyl-2-azabicyclo[2.2.1] hept-5-en-3-on wurden bei 30 °C (+/- 2 °C) inkubiert. Der pH Wert wurde durch manuelle Zugabe von 1 N NaOH bei 7,5 gehalten, wobei Schwankungen von +/- 0,5 pH erreicht wurden. Proben wurden gemäss Beispiel 1.1.1 entnommen. Nach 0,5 h war alles (+) 2-Acetyl-2-azabicyclo[2.2.1] hept-5-en-3-on umgesetzt. Der Enantiomerenüberschuss für (-) 2-Acetyl-2-azabicyclo[2.2.1] hept-5-en-3-on war >98%. Die analytische Ausbeute bezogen auf (-) 2-Acetyl-2-azabicyclo[2.2. 1] hept-5-en-3-on war > 40% bez. des Racemats. Gehalt und Enantiomerenüberschuss wurden wie in Beispiel 1.1.1 beschrieben bestimmt.
**1.1.7.2** 125 mg Aspergillus oryzae (Sigma P-4032, 3.5 Units/mg), 2,25 ml Phosphat-Puffer (100 mM, pH 7,5), 2,5 ml n-Propanol und 0,25 ml (+/-) 2-Acetyl-2-azabicyclo[2.2.1] hept-5-en-3-on (Gehalt: 95,4%) wurden bei 30 °C (+/- 2 °C) inkubiert. Der pH Wert wurde durch manuelle Zugabe von 1 N NaOH bei 7,5 gehalten, wobei Schwankungen von +/- 0,5 pH erreicht wurden. Proben wurden gemäss Beispiel 1.1.1 entnommen. Nach 0,5 h war alles (+) 2-Acetyl-2-azabicyclo[2.2.1] hept-5-en-3-on umgesetzt. Der Enantiomerenüberschuss für (-) 2-Acetyl-2-azabicyclo[2.2.1] hept-5-en-3-on war >98%. Die analytische Ausbeute bezogen auf(-) 2-Acetyl-2-azabicyclo[2.2.1] hept-5-en-3-on war > 40% bez. des Racemats.

### 1.1.8 mittels Proteinase K von Tritirachium album in Phosphat-Puffer

**1.1.8.1** 25 mg Proteinase K (Sigma P-8044 1 - 7 Units/mg), 0,45 ml Phosphat-Puffer (100 mM, pH 7,5), 0,5 ml n-Propanol und 0,05 ml (+/-) 2-Acetyl-2-azabicyclo[2.2.1] hept-5-en-3-on wurden bei 30 °C (+/- 2 °C) inkubiert. Der pH Wert wurde durch manuelle Zugabe von 1 N NaOH bei 7,5 gehalten, wobei Schwankungen von +/- 0,5 pH erreicht wurden. Proben wurden gemäss Beispiel 1.1.1 entnommen. Nach 0,5 h war alles (+) 2-Acetyl-2-azabicyclo[2.2.1] hept-5-en-3-on umgesetzt. Der Enantiomerenüberschuss für (-) 2-Acetyl-2-azabicyclo[2.2.1] hept-5-en-3-on war >98%. Die analytische Ausbeute bezogen auf (-) 2-Acetyl-2-azabicyclo[2.2.1] hept-5-en-3-on war 40 % bez. des Racemats. Gehalt und Enantiomerenüberschuss wurden wie in Beispiel 1 beschrieben bestimmt.
**1.1.8.2** 125 mg Proteinase K (Sigma P-8044 1 - 7 Units/mg), 2,25 ml Phosphat-Puffer (100 mM, pH 7,5), 2,5 ml n-Propanol und 0,25 ml (+/-) 2-Acetyl-2-azabicyclo[2.2.1] hept-5-en-3-on (Gehalt: 95,4%) wurden bei 30 °C (+/- 2 °C) inkubiert. Der pH Wert wurde durch manuelle Zugabe von 1 N NaOH bei 7,5 gehalten, wobei Schwankungen von +/- 0,5 pH erreicht wurden. Proben wurden gemäss Beispiel 1.1.1 entnommen. Nach 0,5 h war alles (+) 2-Acetyl-2-azabicyclo[2.2.1] hept-5-en-3-on umgesetzt. Der Enantiomerenüberschuss für (-) 2-Acetyl-2-azabicyclo[2.2.1] hept-5-en-3-on war >98%. Die analytische Ausbeute bezogen auf (-) 2-Acetyl-2-azabicyclo[2.2.1] hept-5-en-3-on war 30 % bez. des Racemats.

### 1.2 Herstellung von (1S,4R)-2-Acetyl-2-azabicyclo[2.2.1]hept-5-en-3-on (mittels Lipase von Candida antarctica)

**1.2.1** 25 mg SP525 (Novo Nordisk), 0,45 ml Phosphat-Puffer (100 mM, pH 7,5), 0,5 ml n-Propanol und 0,05 ml (+/-) 2-Acetyl-2-azabicyclo[2.2.1] hept-5-en-3-on wurden bei 30 °C (+/- 2 °C) inkubiert. Der pH Wert wurde durch manuelle Zugabe von 1 N NaOH bei 7,5 gehalten, wobei Schwankungen von +/- 0,5 pH erreicht wurden. Proben wurden gemäss Beispiel 1.1.1 entnommen. Nach 0,5 h war alles (-) 2-Acetyl-2-azabicyclo[2.2.1] hept-5-en-3-on umgesetzt. Der Enantiomerenüberschuss für (-) (1S,4R)-2-Acetyl-2-azabicyclo[2.2.1] hept-5-en-3-on war >98%. Die analytische Ausbeute bezogen auf (+) 2-Acetyl-2-azabicyclo[2.2.1] hept-5-en-3-on war 12%. Gehalt und Enantiomerenüberschuss wurden wie in Beispiel 1 beschrieben bestimmt.
**1.2.2** 250 mg SP525 (Novo Nordisk), 2,25 ml Phosphat-Puffer (100 mM, pH 7,5), 2,5 ml n-Propanol und 2,25 ml (+/-) 2-Acetyl-2-azabicyclo[2.2.1] hept-5-en-3-on (Gehalt: 95,4%) wurden bei 30 °C (+/- 2 °C) inkubiert. Der pH Wert wurde durch manuelle Zugabe von 1 N NaOH bei 7,5 gehalten, wobei Schwankungen von +/- 0,5 pH erreicht wurden. Proben wurden gemäss Beispiel 1.1.1 entnommen. Nach 16 h war alles (-) 2-Acetyl-2-azabicyclo[2.2.1] hept-5-en-3-on umgesetzt. Der Enantiomerenüberschuss für (+) (1S,4R)-2-Acetyl-2-azabicyclo[2.2.1] hept-5-en-3-on war >98%. Die analytische Ausbeute bezogen auf (+) 2-Acetyl-2-azabicyclo[2.2.1] hept-5-en-3-on war 12% bez. des Racemats.

### 1.3. Reduktion von (1R,4S)-2-Acetyl-2-azabicylo[2.2.1] hept-5-en-3-on zu (1R,4S)-1-Acetylamino-4-(hydroxymethyl)-2-cyclopenten

**1.3.1** Zu einer Lösung von (1R,4S)-2-Acetyl-2-azabicylo[2.2.1] hept-5-en-3-on (50 g, 0,33 mol), 40 ml Wasser, 240 ml 2-Butanol wurden portionsweise 8 g NaBH₄ zugegeben und die Temperatur unter 5°C gehalten. Nach 1 h wurde die Reaktion gestoppt und dann die Reaktionsmischung mit HCl konz. auf pH 2,0 eingestellt. Die Reaktionstemperatur wurde unter 10°C gehalten. Der pH-Wert wurde mit 30%-iger NaOH auf pH 9,0 eingestellt. Natriummetaborat wurde abfiltriert und die Wasserphase wurde dreimal mit 2-Butanol extrahiert. Nach Abdampfen von 2-Butanol wurden 49,3 g Produkt (0,28 mol) entsprechend einer Ausbeute von 88% erhalten.
**1.3.2.** 287,4 g (-)-Acetyl-2-azabicyclo[2.2.1]hept-5-en-3-on (100 %-ig ⇒ ∼ 255 ml, 97%-ig; 1,9 mol) wurden in 380 ml Wasser und 1217 ml 2-Butanol gelöst. Die Lösung wurde auf 0 bis - 2 °C gekühlt. In einem anderen Rührwerk wurden 45 g NaBH₄ (1,188 mol, 1,25 Eq) in 304 ml frisches 2-Butanol suspendiert. Die NaBH₄-Suspension wurde innerhalb 1 - 2 Stunden in die Lösung zugegeben. Die Reaktion war exotherm und die Temperatur durfte 5 °C nicht überschreiten. Vor einer Portionzugabe musste die Temperatur bei 0 °C liegen. Die Reaktion wurde mittels DC (Hexan/Etrol/MeOH : 5/5/1) verfolgt. Nach der Zugabe wurde noch 1 bis 2 h nachreagieren gelassen. Der Umsatz wurde kontrolliert. Wenn der Umsatz in Ordnung war, (Konzentration an Edukt sollte < 1,0% sein) wurde mit ca. 135 g konz. Salzsäure auf pH 2 eingestellt. Die Temperatur wurde unter 10 °C gehalten. Dann wurde sofort mit ca. 85 ml 30%-ige Natronlauge aufpH 9 eingestellt. Die ausgefallenen Salze wurden filtriert und mit 127 ml frischem 2-Butanol gewaschen. Das Filtrat und das "Wasch-2-Butanol" wurden gesammelt und die Phasen getrennt. Die Wasserphase wurde noch zweimal mit je 380 ml frischem 2-Butanol extrahiert. Die 2-Butanol-Phasen wurden gesammelt. Man erhielt ca. 2450 g 10%-ige Lösung Produkt, (1R,4S)-2-Acetyl-1-amino-4-(hydroxymethyl)-2-cyclopenten in 2-Butanol. Dies entsprach ca. 250 g 100%-iges Produkt (1R,4S)-1-Acetylamino-4-(hydroxymethyl)-2-cyclopenten, entsprechend einer Ausbeute von 85%.

### 1.4. Hydrolyse von (1R,4S)-1-Acetylamino-4-(hydroxymethyl)-2-cyclopenten zu (1R,4S)-1-Amino-4-(hydroxymethyl)-2-cyclopenten

**1.4.1** Zu 49,3 g (0,28 mol) (1R,4S)-1-Acetylamino-4-(hydroxymethyl)-2-cyclopenten wurde 30%ige NaOH (45 g) zugegeben und die Suspension auf 100°C erwärmt. Nach 3,5 h wurde die Lösung auf 0°C abgekühlt und dann mit konz. HCl auf pH = 1,0 eingestellt. Dann wurde Wasser abgedampft und NaCl abfiltriert. Anschtiessend wurde Pentanol (2 ml pro g Rest) und Aceton (6 ml pro g Rest) hinzugegeben und das ausgefallene Präzipitat filtriert und mit 20 ml Aceton gewaschen. Es wurden 37,5 g (0,24 mol) Produkt als Hydrochloridsalz mit einem ee = 99%, entsprechend einer Ausbeute von 86% erhalten.
**1.4.2.** 85,4 g (1R,4S)-(-)-1-Acetylamino-4-(hydroxymethyl)-2-cyclopenten 100 %-ig (0,55 mol) als 10%-ige Lösung in 2-Butanol wurden vorgelegt. Es wurde dann destilliert bis kein Destillat mehr kommt. Anschliessend wurden 110,0 g 30%-ige Natronlauge (⇒ 33,0 g NaOH 100%-ig; 0,825 mol; 1,5 Eq) und 65 g Wasser zugegeben. Das restliche 2-Butanol wurde durch azeotrope Destillation entfernt (mit ca. 10 g Wasser). Dann wurde die Lösung unter Rückfluss (100 - 110 °C) für 4 - 5 h erhitzt. Die Reaktion wurde mittels GC verfolgt. Wenn der Umsatz in Ordnung war, wurde auf 50 °C abgekühlt und 154 ml 2-Butanol (124,3 g) wurden zugegeben. Die Phasen wurden bei 50 °C getrennt und die Wasserphase nochmals mit 154 ml 2-Butanol (124,3 g) bei 50 °C extrahiert (15 Min Rühren, Phasen trennen). Die Wasserphase (ca. 165 g) wurde entsorgt. Die organischen Phasen wurden gesammelt und ca. 22 g Chlorwasserstoff wurden bei 20 - 40 °C bis pH 1 zugegeben. Einige Salze fielen während des Ansäuerns aus. Diese Salze wurden bei 20 °C filtriert und das Filtrat wurde unter Normaldruck destilliert bis 220 ml Destillat (ca. 180 g) aufgefangen waren (Kochtemperatur ca. 91 - 92 °C). Danach wurden bei ca. 70 °C 176 ml Aceton (139,0 g) zugegeben. Die Suspension wurde 15 bis 30 min unter Rückfluss gerührt und dannach auf -5 °C abgekühlt. Nach 1 h bei dieser Temperatur wurde die Suspension abgenutscht und der Filterkuchen mit 154 ml Aceton gewaschen. Man erhielt 70,0 g (-)-Produkt 100%-ig entsprechend einer Ausbeute von 85%.
Gehalt: 99.0% (Tit., wt.%)
NaCl: 0.5 bis 1.0%

### Beispiel 2

### Herstellung von (1R,4S)-2-Ethoxycarbonyl-2-azabicyclo[2.2.1]hept-5-en-3-on

### 2.1. Herstellung von racemischem (±)-2-Ethoxycarbonyl-2-azabicyclo[2.2.1]hept-5-en-3-on

109,13 g racemisches 2-Azabicyclo[2.2.1]hept-5-en-3-on wurden mit 182,1 g Triethylamin, 6,11 g 4-Dimethylaminopyridin und 500 ml Acetonitril gemischt. Die Reaktion wurde auf 50 °C erwärmt. Danach wurde portionenweise 195,3 g Ethylchlorformiat, gelöst in 150 ml Acetonitril, zugegeben. Die Temperatur wurde unter 55 °C gehalten. Nach Reaktionsende wurde die Lösung auf 20 °C gekühlt und die Salze wurden abfiltriert und mit Acetonitril gewaschen. Das Filtrat wurde bei 60 °C und 20 mbar eingeengt und anschliessend in 1500 ml Toluol aufgenommen. Darauf folgten 3 Extraktionen: mit 250 ml Wasser, pH 8, mit 250 ml Essigsäure (1%), mit 250 ml gesättigter NaCl-Lösung. Die organische Phase wurde mit MgSO₄ getrocknet und bei 80 °C/20 mbar eingeengt. 167,4 g eines braunen Öles wurden erhalten. Der Gehalt nach GC war 96 %. (±)-2-Ethoxycarbonyl-2-azabicyclo[2.2.1]hept-5-en-3-on wurde gereinigt durch Vakuumdestillation, b.p_{0.01} = 76,5 °C, Gehalt (GC) : 99,5 %, Ausbeute : 156,6 g (88,5 %)
¹H-NMR (CDCl₃):
1,33 (t, 3H);
2,19 (d, 1H);
2,38 (d, 1H);
3,43 (s, 1H);
4,26 (m, 2H);
5,04 (s, 1H);
6,68 (m, 1H);
6,92 (m;1H).

### 2.2 Herstellung von (-)-2-Ethoxycarbonyl-2-azabicyclo[2.2.1]hept-5-en-3-on

Analog zu Beispiel 2.1. wurde das Produkt ausgehend von (1R,4S)-(-)-2-Azabicylclo[2.2.1]hept-5-en-3-on hergestellt.

### 2.3 Herstellung von (1R,4S)-2-Ethoxycarbonyl-2-azabicyclo[2.2.1]hept-5-en-3-on

500 µl Savinase gemäss Beispiel 1.1.1, 5 ml n-Propanol, 4,5 ml (50 mM Phosphatpuffer pH 8/Tetrahydrofuran, 1/1) und 250 µl (+/-) Ethoxycarbonyl-2-azabicyclo[2.2.1]hept-5-en-3-on wurden bei 40 °C bei pH 8 inkubiert. Durch manuelle Zugabe von 1N NaOH wurde der pH Wert auf 8 gehalten. Proben wurden wie in Beispiel 1.1.1 entnommen. Nach 4,5 h wurde ein ee Wert von >98 % für das (-) Enantiomere gemessen, was anhand des hergestellten Standards (Beispiel 2.2) bewiesen wurde. Die nichtisolierte, analytische Ausbeute bezogen auf das Racemat betrug 46%.

## Patentansprüche

1. Verfahren zur Herstellung von optisch aktiven Verbindungen der allgemeinen Formeln 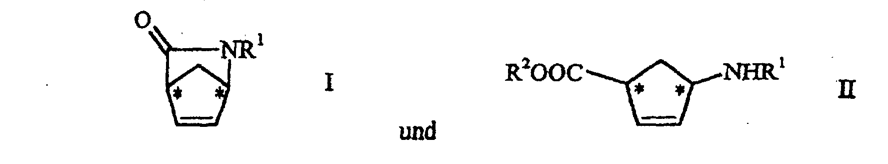 worin R¹ C₁₋₄-Alkanoyl, das mit ein oder mehreren Halogenatomen substituiert ist, Phenylcarbonyl, Benzykarbonyl, Methoxycarbonyl, Ethoxycarbonyl, Propoxycarbonyl, Butoxycarbonyl oder Phenoxycarbonyl bedeutet und R² ein Wasserstoffatom bedeutet, worin ein racemisches Lactam der allgemeinen Formel 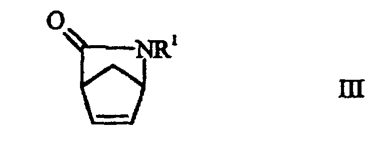 worin R¹ die oben genannte Bedeutung hat, mittels einer Hydrolase in Gegenwart von Wasser als Nucleophil bei einem innerhalb +/- 0,5 Einheiten durch Zugabe einer Base konstant gehaltenen pH-Wert umgesetzt wird.

2. Verfahren nach Anspruch 1, **dadurch gekennzeichnet, dass** die Umsetzung in einer Pufferlösung durchgeführt wird.

3. Verfahren nach Anspruch 1 oder 2, **dadurch gekennzeichnet, dass** das Nucleophil in einem Gemisch mit einem aprotischen organischen Lösungsmittel eingesetzt wird.

4. Verfahren zur Herstellung von optisch aktiven Verbindungen der allgemeinen Formeln 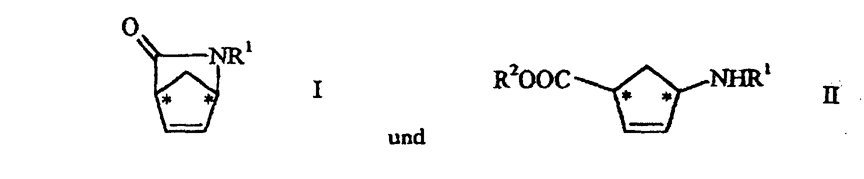 worin R¹ Acyl, Alkoxycarbonyl oder Aryloxycarbonyl bedeutet und R² C₁₋₁₀-Alkyl bedeutet, worin ein racemisches Lactam der allgemeinen Formel 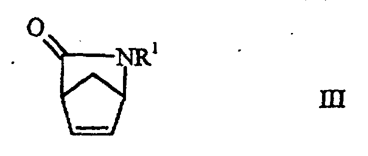 worin R¹ die genannte Bedeutung hat, mittels einer Hydrolase in Gegenwart eines C₁₋₁₀-Alkohols als Nucleophil bei einem innerhalb +/- 0,5 Einheiten durch Zugabe einer Base konstant gehaltenen pH-Wert umgesetzt wird.

5. Verfahren nach Anspruch 4, **dadurch gekennzeichnet, dass** der C₁₋₁₀-Alkohol in einem Gemisch mit einem aprotischen organischen Lösungsmittel eingesetzt wird.

6. Verfahren nach irgendeinem der Ansprüche 1 bis 5, **dadurch gekennzeichnet, dass** als Hydrolase eine Protease oder Lipase verwendet wird.

7. Verfahren nach Anspruch 6, **dadurch gekennzeichnet, dass** als Protease eine Serinprotease verwendet wird.

8. Verfahren nach Anspruch 7, **dadurch gekennzeichnet, dass** als Serinprotease ein Subtilisin verwendet wird.

9. Verfahren nach irgendeinem der Ansprüche 1 bis 8, **dadurch gekennzeichnet, dass** als racemisches Lactam der allgemeinen Formel III 2-Acetyl-2-azabicyclo[2.2.1]hept-5-en-3-on oder 2-Ethoxycarbonyl-2-azabicyclo[2.2.1]hept-5-en-3-on eingesetzt wird.

10. Verfahren nach irgendeinem der Ansprüche 1 bis 9, **dadurch gekennzeichnet, dass** die Umsetzung bei einer Temperatur von 10 bis 60°C durchgeführt wird.

11. Verfahren zur Herstellung von optisch aktiven 1-Amino-4-(hydroxymethyl)-2-cyclopenten-Derivaten der allgemeinen Formel 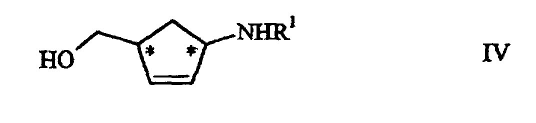 worin R¹ Acyl, Alkoxycarbonyl oder Aryloxycarbonyl bedeutet, **dadurch gekennzeichnet, dass** ein Lactam der allgemeinen Formel 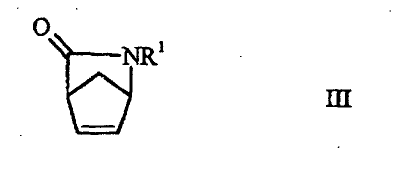 worin R¹ die genannte Bedeutung hat, mittels einer Hydrolase in Gegenwart eines C₁₋₁₀-Alkohols als Nucleophil bei einem innerhalb +/- 0,5 Einheiten durch Zugabe einer Base konstant gehaltenen pH-Wert in die Verbindung der allgemeinen Formel 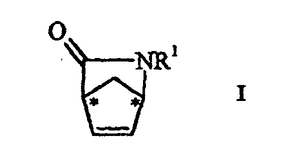 worin R¹ die genannte Bedeutung hat, überführt wird und diese dann zu der Verbindung der allgemeinen Formel IV reduziert wird.

12. Verfahren zur Herstellung von (1R,4S)-1-Amino-4-(hydroxymethyl)-2-cyclopenten der Formel 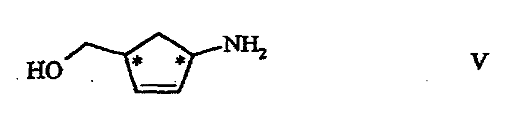 bzw. dessen Salzen, **dadurch gekennzeichnet, dass** ein Lactam der allgemeinen Formel 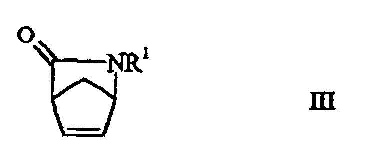 worin R¹ Acyl, Alkoxycarbonyl oder Aryloxycarbonyl bedeutet, mittels einer Hydrolase in Gegenwart eines Nucleophils bei einem innerhalb +/- 0,5 Einheiten durch Zugabe einer Base konstant gehaltenen pH-Wert in die Verbindung der allgemeinen Formel 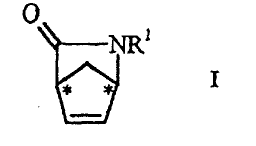 worin R¹ die genannte Bedeutung hat, überführt wird, diese dann zur Verbindung . der allgemeinen Formel 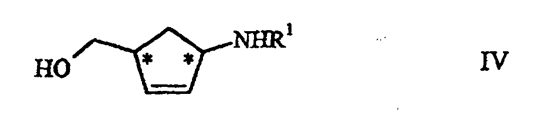 worin R¹ die genannte Bedeutung hat, reduziert wird und diese dann zu der Verbindung der Formel V hydrolysiert wird.

13. (1S,4R)-4-Acetylamino-2-cyclopenten-1-carbonsäure-C₂₋₁₀-alkylester.

14. (1S,4R)-4-Acetylamino-2-cyclopenten-1-carbonsäureethylester.

15. (1S,4R)-4-Acetylamino-2-cyclopenten-1-carbonsäurepropylester.

## Claims

1. Method for preparing optically active compounds of the general formulas 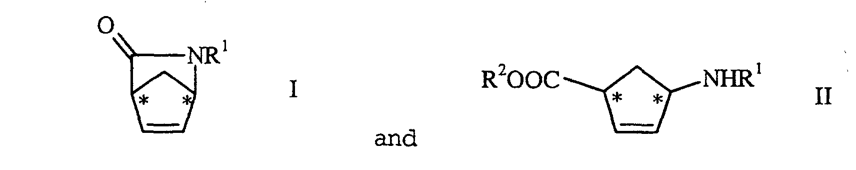 wherein R¹ is C₁₋₄ alkanoyl which is substituted by one or more halogen atoms, phenylcarbonyl, benzylcarbonyl, methoxycarbonyl, ethoxycarbonyl, propoxycarbonyl, butoxycarbonyl or phenoxycarbonyl and R² is a hydrogen atom, wherein a racemic lactam of the general formula 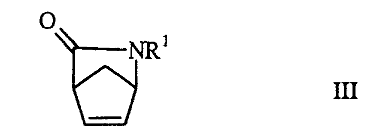 wherein R¹ is as defined above, is converted by means of a hydrolase in the presence of water as a nucleophile at a pH value maintained constant at +/- 0.5 pH units by addition of a base.

2. Method according to Claim 1, **characterized in that** the conversion is conducted in a buffer solution.

3. Method according to Claim 1 or 2, **characterized in that** the nucleophile is used in a mixture with an aprotic organic solvent.

4. Method for preparing optically active compounds of the general formulas 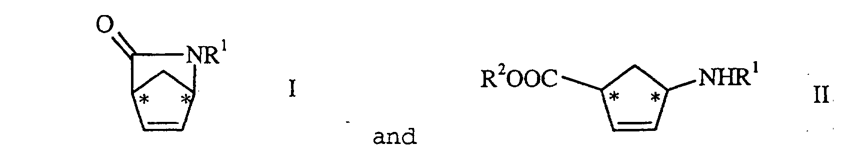 wherein R¹ is acyl, alkoxycarbonyl or aryloxycarbonyl and R² is C₁₋₁₀ alkyl, wherein a racemic lactam of the general formula 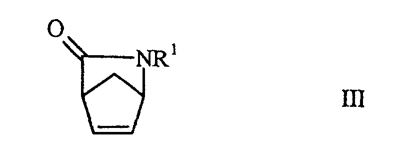 wherein R¹ is as defined above, is converted by means of a hydrolase in the presence of a C₁₋₁₀ alcohol as a nucleophile at a pH value maintained constant at +/- 0.5 pH units by addition of a base.

5. Method according to Claim 4, **characterized in that** the C₁₋₁₀ alcohol is used in a mixture with an aprotic organic solvent.

6. Method according to any one of Claims 1 to 5, **characterized in that** a protease or lipase is used as the hydrolase.

7. Method according to Claim 6, **characterized in that** a serinprotease is used as the protease.

8. Method according to Claim 7, **characterized in that** a subtilisin is used as the serinprotease.

9. Method according to any one of Claims 1 to 8, **characterized in that** 2-acetyl-2-azabicyclo[2.2.1]-hept-5-ene-3-one or 2-ethoxycarbonyl-2-azabicyclo-[2.2.1]hept-5-ene-3-one is used as the racemic lactam of the general formula III.

10. Method according to any one of Claims 1 to 9, **characterized in that** the conversion is conducted at a temperature of from 10 to 60°C.

11. Method for preparing optically active 1-amino-4-(hydroxymethyl)-2-cyclopentene derivatives of the general formula 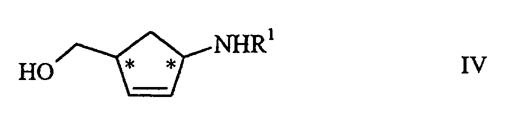 wherein R¹ is acyl, alkoxycarbonyl or aryloxycarbonyl, **characterized in that** a lactam of the general formula 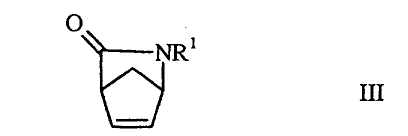 wherein R¹ is as defined above, is converted by means of a hydrolase in the presence of a C₁₋₁₀ alcohol as a nucleophile at a pH value maintained constant at +/- 0.5 pH units by addition of a base to give the compound of the general formula 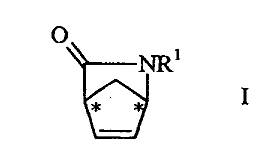 wherein R¹ is as defined above, and this is then reduced to give the compound of the general formula IV

12. Method for preparing (1R,4S)-1-amino-4-(hydroxymethyl)-2-cyclopentene of the formula 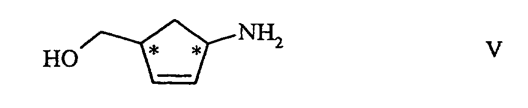 or its salts, **characterized in that** a lactam of the general formula 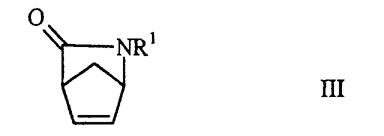 wherein R¹ is acyl, alkoxycarbonyl or aryloxycarbonyl, is converted by means of a hydrolase in the presence of a nucleophile at a pH value maintained constant at +/- 0.5 pH units by addition of a base to give the compound of the general formula 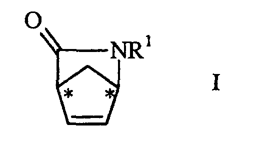 wherein R¹ is as defined above, this is then reduced to give the compound of the general formula IV 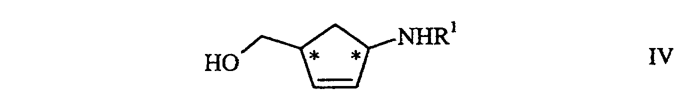 wherein R¹ is as defined above, and this is then hydrolyzed to give the compound of formula V.

13. (1S,4R)-4-Acetylamino-2-cylopentene-1-carboxylic acid C₂₋₁₀-alkylester.

14. (1S,4R)-4-Acetylamino-2-cylopentene-1-carboxylic acid ethylester.

15. (1S,4R)-4-Acetylamino-2-cylopentene-1-carboxylic acid propylester.

## Revendications

1. Procédé de préparation de composés optiquement actifs de formules générales : 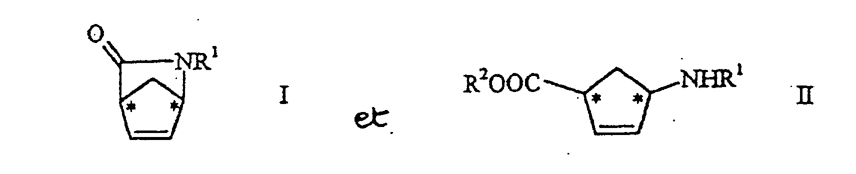 où R¹ représente un alcanoyle en C₁₋₄, qui est substitué par un ou plusieurs atomes d'halogène, phénylcarbonyle, benzylcarbonyle, méthoxycarbonyle, éthoxycarbonyle, propoxycarbonyle, butoxycarbonyle ou phénoxycarbonyle et R² représente un atome d'hydrogène, où un lactame racémique de formule générale : 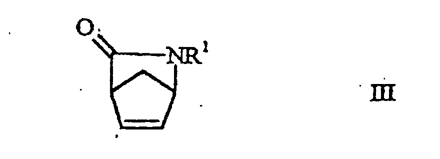 où R¹ a la signification indiquée ci-dessus, est converti à l'aide d'une hydrolase en présence d'eau comme nucléophile à un pH maintenu constant dans un intervalle de +/- 0,5 unité par addition d'une base.

2. Procédé selon la revendication 1, **caractérisé en ce que** la conversion est réalisée dans une solution tampon.

3. Procédé selon la revendication 1 ou 2, **caractérisé en ce que** le nucléophile est mis en oeuvre en mélange avec un solvant organique aprotique.

4. Procédé de préparation de composés optiquement actifs de formules générales : 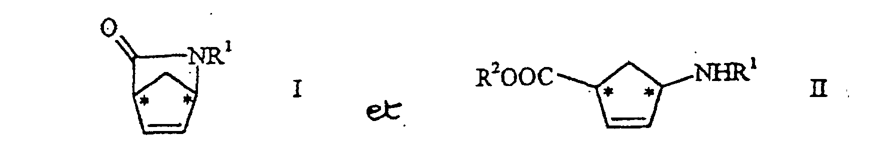 où R¹ représente un acyle, un alcoxycarbonyle ou un aryloxycarbonyle, et R² représente un alkyle en C₁₋₁₀, où un lactame racémique de formule générale : 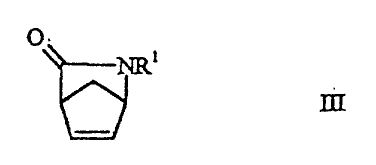 où R¹ a la signification indiquée ci-dessus, est converti à l'aide d'une hydrolase en présence d'un alcool en C₁₋₁₀ comme nucléophile à un pH maintenu constant dans un intervalle de +/- 0,5 unité par addition d'une base.

5. Procédé selon la revendication 4, **caractérisé en ce que** l' alcool en C₁₋₁₀ est mis en oeuvre en mélange avec un solvant organique aprotique.

6. Procédé selon l'une quelconque des revendications 1 à 5, **caractérisé en ce que** l'on utilise comme hydrolase, une protéase ou une lipase.

7. Procédé selon la revendication 6, **caractérisé en ce que** l'on utilise comme protéase, une sérine protéase.

8. Procédé selon la revendication 7, **caractérisé en ce que** l'on utilise comme sérine protéase, une subtilisine.

9. Procédé selon l'une quelconque des revendications 1 à 8, **caractérisé en ce que** l'on met en oeuvre comme lactame racémique de formule générale III, la 2-acétyl-2-azabicyclo[2.2.1]hept-5-ène-3-one ou la 2-éthoxycarbonyl-2-azabicyclo[2.2.1]hept-5-ène-3-one.

10. Procédé selon l'une quelconque des revendications 1 à 9, **caractérisé en ce que** la conversion est réalisée à des températures allant de 10 à 60°C.

11. Procédé de préparation de dérivés 1-amino-4-(hydroxyméthyl)-2-cyclopentène optiquement actifs de formule générale : 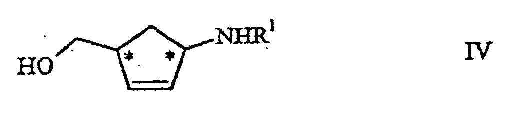 où R¹ représente un acyle, un alcoxycarbonyle ou un aryloxycarbonyle, **caractérisé en ce qu'**un lactame de formule générale : 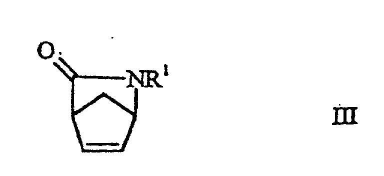 où R¹ a la signification indiquée ci-dessus, est converti à l'aide d'une hydrolase en présence d'un alcool en C₁₋₁₀ comme nucléophile à un pH maintenu constant dans un intervalle de +/- 0,5 unité par addition d'une base, en composé de formule générale : 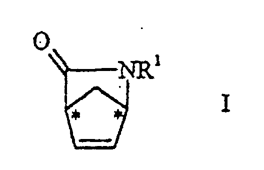 où R¹ a la signification indiquée ci-dessus, et celui-ci est alors réduit en composé de formule générale IV.

12. Procédé de préparation du (1R,4S)-1-amino-4-(hydroxyméthyl)-2-cyclopentène de formule : 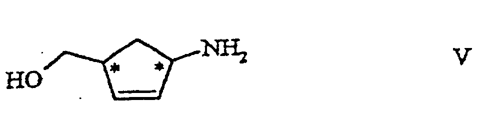 ou ses sels, **caractérisé en ce qu'**un lactame de formule générale : 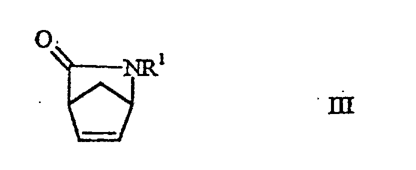 où R¹ représente un acyle, un alcoxycarbonyle ou un aryloxycarbonyle, est converti à l'aide d'une hydrolase en présence d'un nucléophile à un pH maintenu constant dans un intervalle de +/- 0,5 unité par addition d'une base, en composé de formule générale : 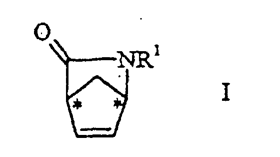 où R¹ a la signification indiquée ci-dessus, et celui-ci est alors réduit en composé de formule générale IV : 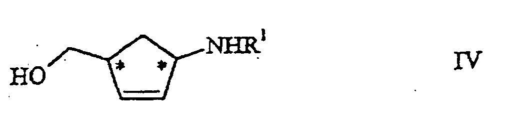 où R¹ a la signification indiquée ci-dessus, et ce dernier est alors hydrolysé en composé de formule V.

13. Ester alkylique en C₂₋₁₀ de l'acide (1S,4R)-4-acétylamino-2-cyclopentèn-1-carboxylique.

14. Ester éthylique de l'acide (1S,4R)-4-acétylamino-2-cyclopentèn-1-carboxylique.

15. Ester propylique de l'acide (1S,4R)-4-acétylamino-2-cyclopentèn-1-carboxylique.
